# EUROPEAN PATENT APPLICATION

(11) **EP 4 344 618 A1**
(43) Date of publication of application: **03.04.2024**
(21) Application number: 22197946.1
(22) Date of filing: 27.09.2022
(51) Int. Cl.: A61B 5/00, A61B 5/0533, A61B 5/024, A61B 5/08

(54) **PAIN MONITORING**

(71) Applicant: Koninklijke Philips N.V., 5656 AG Eindhoven (NL)
(72) Inventor: RMAILE, Amir Hussein, Eindhoven (NL); JOHNSON, Mark Thomas, Eindhoven (NL); GERHARDT, Lutz Christian, Eindhoven (NL); SPELT, Hanne Adriana Alijda, Eindhoven (NL); KOOIJMAN, Gerben, Eindhoven (NL); PERRONE, Antonio Luigi, Eindhoven (NL)
(74) Representative: Philips Intellectual Property & Standards

(57) **Abstract**

A method for tracking patient recovery status following a treatment or consultation. The method is based on capturing sensor data during personal care sessions of a user, and using this to infer user pain information. For example, pain can be inferred from a biological sensor or from patterns in force/motion sensors integrated in the personal care device. The pain data can be converted into a value of a metric for each personal care session, and by trending this metric over time, patient recovery progress can be inferred. A report can be generated based on the trended metric and exported for example to a clinical practitioner.

## Description

### FIELD OF THE INVENTION

This invention relates to a method and system for monitoring pain, for example following a treatment or a consultation.

### BACKGROUND OF THE INVENTION

Following a dental treatment (e.g. tooth extraction, pocket cleaning, scaling, root canal, oral surgery), the dental practitioner has little or no way to monitor the healing process of the patient. The patient may believe that any experienced pain post-treatment is normal and thus not alert the dental practitioner, whereas such pain could instead be a sign of problems. As a result, problems may be missed, leading to medical complications or a worse outcome. This is detrimental for the patient and also for the dental practitioner and damages the dentist-patient relationship.

In a survey conducted by the applicant, in which 45 dental professionals were interviewed, a top highlighted concern was the absence of information about the progress of patient recovery after treatment.

The same problem also applies to monitoring patient status following a dental consultation at which the patient reported pain, but no treatment was given. The dentist might ask the patient to monitor the condition and get back in touch in the event that the pain gets worse or does not abate. The patient might lose track of the progress of their pain, may acclimatize to the pain and so begin to ignore it, and thus not follow up with the dental practitioner to book a follow up appointment. This can lead to an initially minor problem becoming worse, with detrimental consequences for the patient's dental health.

A similar problem can likewise exist outside of the oral health domain, for instance related to skin health status in the area of dermatology. A dermatologist may provide treatment for skin, but has no way to track progress of the condition subsequently. Another example is the area of muscular joint pain, where improvement is hard to monitor without regular physical examinations.

A means to facilitate easier and more objective monitoring of a patient status post treatment or post consultation would therefore be of value.

### SUMMARY OF THE INVENTION

The invention is defined by the claims.

According to examples in accordance with an aspect of the invention, there is provided a method for determining a status (e.g. a recovery status) of a patient following a treatment or consultation. The method comprises: obtaining data indicative of user pain as a function of time over the course of each of a series of personal care sessions by a user using a personal care device, the personal care sessions following the treatment or consultation, and wherein the data indicative of user pain is determined based on sensor data from at least one sensor; determining a metric for each session based on the data indicative of user pain; computing a post-treatment or post-consultation status based on trending of the metric over the series of personal care sessions following the treatment, and generating a data report for export indicative of the computed post-treatment or post-consultation status.

It is proposed to monitor a patient post treatment or post-consultation via capturing sensor data during their normal home personal care routines (for example oral cleaning routines, e.g. when brushing their teeth, or grooming routines, e.g. when shaving). Sensor data is captured which is indirectly indicative of pain and then this is used to compute a metric for each session, and the values of this metric can then be tracked over time to monitor patient status. The concept is based on the realization that systematic use of a personal care device is something that a user routinely does at regular and reliable time points, and that its normal use typically entails application of a systematic series of physical stimuli to an anatomy or different areas of an anatomy. This therefore provides an efficient way of measuring pain information associated with the relevant anatomy, and using data captured from sensors associated with the personal care device. Thus a personal care session at home provides a convenient opportunity to obtain sensor data which provides an indication of pain level, and that pain level provides an indication of recovery status, or of the progress of improvement of a pathology or wound. For example, when a user is cleaning their teeth, they naturally apply a systematic series of pressure stimuli around the mouth, which therefore stimulates pain responses in areas where there is a dental problem, and the sensor data measurement can be used to detect this. Likewise when shaving, stimuli are applied to different areas of the skin.

One application area is in the domain of oral care. In some embodiments, the personal care device is an oral care device, the aforementioned personal care action is cleaning action, and the personal care session is an oral cleaning session. The consultation in this case may be a dental consultation and/or the treatment may be a dental treatment. In further embodiments, the personal care device may be a skin care device, e.g. a light therapy device, and the personal care action is skin care action, and the personal care session is a skin care session. In further embodiments, the personal care device is a grooming device, e.g. a shaver, and the personal care action is a grooming action (e.g. shaving), and the personal care session is a grooming session (e.g. shaving). In further embodiments, the personal care device is a muscle/joint care device (e.g. a massage device). In each case, the personal care device is a device for application to an area of the user's body for a care function, whereby physical stimulation of one or more areas of the body is caused by such application during the care session. Thus, the normal use of the device inherently entails a systematic physical probing of one or more areas of the body, whereby a pain response may be elicited and can be measured through monitoring patterns in the sensor data.

With regards to the metric computed for each personal care session, this metric may in some embodiments comprise an overall pain experience metric for each session. It may comprise an average value of one or more sensor readings over the personal care session, where the one or more sensor readings are themselves indirectly indicative of user pain. For example, the metric could be an average value for one or more sensor measurements which provide a proxy for pain, such as applied pressure.

There are different options for the sensor data based upon which the data indicative of user pain is determined.

In some embodiments, the sensor data includes data from a biological sensor. For example the biological sensor may be a skin conductance sensor, such as a galvanic skin response (GSR) sensor. Such sensor data is known to reflect pain-related arousal, and thus can be used to obtain user pain information. Other example biological sensors include a heart rate sensor, a PPG sensor, a muscle tension sensor, or a camera to sense facial expression.

In some embodiments, a biological sensor could be integrated in the personal care device, for example integrated in a handle of the device. In other examples, a biological sensor could be provided as a separate sensor unit.

Additionally or alternatively to use of a biological sensor, in some embodiments, the sensor data includes data from one or more force and/or motion sensors integrated in the personal care device indicative of personal care action by the user using the device over the course of one or more personal care sessions, or from one or more operational signal sensors integrated in the personal care device adapted to output an operational signal indicative of an operational parameter of the device which correlates with physical interaction of the device with bodily surfaces. An example of an operational parameter is an electrical drive signal associated with an actuation device which actuates a portion of the personal care device designed for contacting bodily surfaces during use, e.g. a drive train mechanism which drives oscillation of a body-contacting portion of a powered toothbrush or shaver, or a motor mechanism which drives a body-contacting portion of a massage device. The electrical characteristics of the drive signal can change as a function of applied pressure since applied pressure exerts more physical resistance. Another example of an operational parameter is jet flow of an oral irrigation device or powered flossing device.

The information indicative of user pain during each of the one or more personal care sessions may be determined based on analysis of patterns in the sensor data, and optionally based on reference sensor pattern information associated with user pain or user comfort. This approach will be explained in greater detail to follow.

In some embodiments, the computing the patient status comprises determining, from trending of the metric over the series of personal care sessions, a time duration following the treatment or consultation for which the metric remains above a pre-determined threshold level.

In some embodiments, the method further comprises comparing said time duration following treatment or consultation for which the metric remains above a pre-determined threshold level against a pre-defined expected recovery time period and determining whether the time period exceeds the expected recovery period.

In some embodiments, the threshold level is determined based on a pre-treatment or pre-consultation metric level measured using sensor data from the at least one sensor acquired in advance of the treatment.

In some embodiments, the determining the post-treatment or post-consultation status comprises comparing a rate of change of said metric over the series of sessions following treatment against a threshold rate of change. In other words, it may be checked whether pain is decreasing at the rate expected.

In some embodiments, the method comprises performing a response action based on the patient status. The response action could for example include automatic scheduling of an appointment with the practitioner, e.g. a dental practitioner, or sending an alert message to the practitioner.

In some embodiments, the response action comprises transmitting the data report via a communication channel to a practitioner computing device.

In some embodiments, the data report is only transmitted in the event that the post-treatment or post-consultation status is determined to be outside of a normal range, and optionally wherein the normal range is determined in dependence upon a time elapsed since the treatment event (i.e. the expected recovery status depends upon how long ago the treatment occurred).

In some embodiments, the personal care device is an oral care device, the personal care session is an oral cleaning session and the personal care action is cleaning action. The treatment may be a dental treatment and/or the consultation may be a dental consultation.

Another aspect of the invention is a processing device, comprising: an input/output; and one or more processors. The one or more processors are configured to: obtain information indicative of user pain as a function of time over the course of each of a series of personal care sessions by a user using a personal care device, the personal care sessions following the treatment, and wherein the data indicative of user pain is determined based on sensor data from at least one sensor; determine a metric for each session based on the data indicative of user pain; compute a post-treatment or post-consultation patient status based on trending of said metric over the series of personal care sessions following the treatment or consultation event, and generate a data report for export indicative of the computed post-treatment or post-consultation status.

Another aspect of the invention is a system. The system may comprise a personal care device. In some embodiments, the personal care device comprises one or more force or motion sensors configured to generate sensor data indicative of personal care action by the user using the device over the course of one or more personal care sessions or one or more operational signal sensors integrated in the personal care device adapted to output an operational signal indicative of an operational parameter of the device which correlates with physical interaction of the device with bodily surfaces. In some embodiments, the personal care device comprises a biological sensor configured to generate sensor data indicative of a biological parameter of the user over the course of one or more personal care sessions. The system further comprises a processing device in accordance with any embodiment described in this document, e.g. as outlined above, and wherein the processing device is arranged to receive the generated sensor data as an input at the input/output. In one set of embodiments, the personal care device is an oral care device.

In some embodiments, the processing device is integrated in the personal care device.

In some embodiments, the processing device is comprised by a computing device external to the personal care device, the computing device communicatively coupled with the personal care device or with a datastore with which the personal care device is communicatively coupled.

In some embodiments, the processing device is a cloud-based processing device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments;
Fig. 2 shows components of an example processing device and system in accordance with one or more embodiments;
Figs. 3-4 illustrate trending of an example metric over a plurality of personal care sessions both before and after a treatment or consultation, and illustrate comparative example scenarios in which recovery status improves and in which recovery status does not improve;
Fig. 5 illustrates trending of an example metric over a plurality of personal care sessions both before and after a treatment or consultation and illustrates how a time period taken for the metric to return to a pre-treatment level can be compared against a reference time period to infer a recovery status; and
Figs. 6-8 illustrate approaches to inferring user pain during personal care sessions from analysis of patterns in force and/or motion sensor data obtained from sensors integrated in a personal care device.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for tracking patient status (e.g. recovery or progress status) following a treatment or consultation. It is based on capturing sensor data during personal care sessions of a user and using this to infer user pain information. For example pain can be inferred from a biological sensor or from patterns in force/motion sensors integrated in the personal care device. The pain data can be converted into a value of a metric for each personal care session, and by trending this metric over time, patient recovery or progress can be inferred. A report can be generated based on the trended metric and exported for example to a practitioner.

The concept is based on the principle that systematic use of a personal care device is something that a user routinely does at regular and reliable time points, and that its normal use typically entails application of a systematic series of physical stimuli to an anatomy or different areas of an anatomy. This therefore provides an efficient way of measuring pain information associated with the relevant anatomy, and using data captured from sensors associated with the personal care device.

By way of example, one application area is for use with oral care devices, for example used for oral cleaning. Since tooth cleaning is something that a person usually already does twice a day, the monitoring approach can very easily and efficiently be integrated in a user's normal daily routine, making compliance likely very high.

One advantage of embodiments of the invention is enabling a clinical practitioner (e.g. dental practitioner, dermatologist, etc.) to stay informed about the progress of patient status even while the patient is at home. This leads to several advantages. It leads to improved patient experience by providing peace of mind that their healing is being monitored by their practitioner. Staff experience is improved by providing the practitioner an insight into how the patient condition is progressing (or regressing). Clinical efficiency is also improved by optimizing practitioner time: for example, scheduling of a follow-up appointment can be deferred until pain data indicate that a problem may be present that requires examination. Another benefit is improved health outcomes. For example, post treatment monitoring of healing increases the odds of a successful treatment.

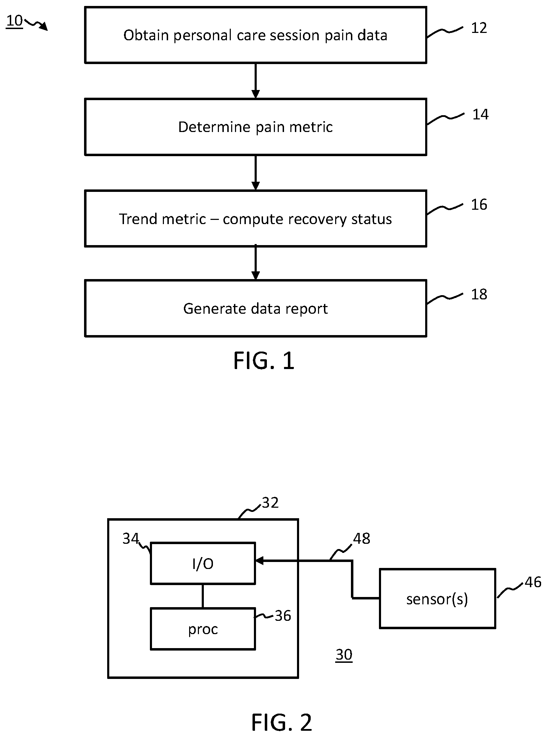

Fig. 1 outlines in block diagram form steps of an example computer implemented method 10 according to one or more embodiments. The steps will be recited in summary, before being explained further in the form of example embodiments.

The method is a method for determining a post-treatment status of a patient following a treatment or consultation.

The method 10 comprises 12 obtaining data indicative of user pain as a function of time over the course of each of a series of personal care sessions by a user using a personal care device. The personal care sessions may be sessions which follow a treatment or consultation. The data indicative of user pain is determined based on sensor data from at least one sensor.

The method further comprises determining 14 a metric for each session based on the data indicative of user pain.

The method further comprises computing 16 a post-treatment or post-consultation status based on trending of the metric over the series of personal care sessions following the treatment or consultation.

The method further comprises generating 18 a data report for export indicative of the computed patient status.

The method may further comprise transmitting the report via a communication channel to a remote computer, for example a computer accessible to a practitioner, e.g. dental practitioner in the case of oral care. For example, the report may be uploaded to a server, for example a cloud-based server or other server which is accessible to the practitioner. This way, the practitioner can be informed as to the recovery status of the patient.

As noted above, the method can also be embodied in hardware form, for example in the form of a processing unit which is configured to carry out a method in accordance with any example or embodiment described in this document, or in accordance with any claim of this application.

To further aid understanding, Fig. 2 presents a schematic representation of an example processing unit 32 configured to execute a method in accordance with one or more embodiments of the invention. The processing unit is shown in the context of a system 30 which comprises the processing unit. The processing unit alone represents an aspect of the invention. The system 30 is another aspect of the invention. The provided system does not have to comprise all of the illustrated hardware components; it may just comprise a subset of them.

The processing unit comprises one or more processors 36 configured to perform a method in accordance with that outlined above, or in accordance with any embodiment described in this document or any claim of this application. In the illustrated example, the processing unit further comprises an input/output 34 or communication interface. In the illustrated example of Fig. 2, the system 30 further comprises one or more sensors 46 for providing sensor data 48.

In some embodiments, the one or more sensors 46 include one or more force or motion sensors configured to generate sensor data indicative of personal care action by the user using the device over the course of one or more personal care sessions. The one or more force or motion sensors may be comprised by a personal care device. The system 30 may comprise the personal care device comprising the one or more force or motion sensors in some embodiments.

In some embodiments, the one or more sensors 46 include one or more operational signal sensors integrated in the personal care device adapted to output an operational signal indicative of an operational parameter of the device which correlates with physical interaction of the device with bodily surfaces. An example of an operational parameter is an electrical drive signal associated with an actuation device which actuates a portion of the personal care device, e.g. a drive train mechanism which drives oscillation of a powered toothbrush or shaver, or a motor mechanism which drives a massage device. The electrical characteristics of the drive signal can change as a function of applied pressure since applied pressure exerts more physical resistance on the actuation mechanism. Another example of an operational parameter is jet flow of an oral irrigation device or powered flossing device.

In some embodiments, the one or more sensors 46 include a biological sensor configured to generate sensor data indicative of a biological parameter of the user over the course of one or more personal care sessions. This may be integrated in a personal care device in some examples or may be separate to any personal care device. The system 30 may comprise a personal care device comprising a biological sensor in some embodiments.

In some embodiments, the processing device 32 is integrated in the personal care device. In further embodiments, the processing device 32 may be comprised by a computing device external to the personal care device, the computing device communicatively coupled with the personal care device or with a datastore with which the personal care device is communicatively coupled. In further embodiments, the processing device 32 may be a cloud-based processing device.

To illustrate and exemplify the inventive concepts, examples are presented below with reference to an oral care device, for which the aforementioned personal care action is cleaning action, and the personal care session is an oral cleaning session. However, it will be recognized that the principles outlined are easily applicable to other types of personal care device, such as, by way of example, a skin care device, e.g. a light therapy device, a grooming device, e.g. a shaver, or a muscle/joint care device (e.g. a massage device). With any personal care device, the device is typically designed for application to an area of the user's body for a care function, whereby physical stimulation of one or more areas of the body is caused by such application during the care session. Thus, the normal use of the device inherently entails a systematic physical probing of one or more areas of the body, whereby a pain response may be elicited and can be measured through the sensor data. Thus it can be seen how the principles of the general inventive concept outlined above, and described below, can be used in any personal care device. Hence, reference in the below-described embodiments to an oral care device may be replaced by reference to any other personal care device without substantive change to the inventive principles. Likewise, reference to cleaning action can be replaced by reference to personal care action and reference to a cleaning session can be replaced by reference to a personal care session.

In the case that the personal care device is an oral care device, the oral care device may be a powered toothbrush in some embodiments. The toothbrush may have a brush head carrying an array of cleaning elements such as bristles.

As mentioned previously, the invention can also be embodied in software form. Thus another aspect of the invention is a computer program product comprising code means configured, when run on a processor, to cause the processor to perform a method in accordance with any example or embodiment of the invention described in this document, or in accordance with any claim of this patent application.

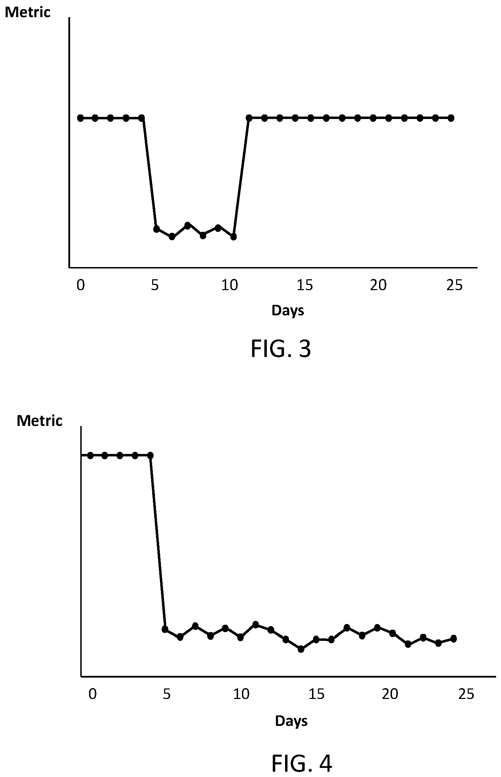

Fig. 3 and Fig. 4 illustrate, by way of simple example, the concept according to one or more embodiments of the invention.

Each graph shows a trend in a metric computed for each of a series of personal care sessions spanning a period of 25 days, wherein the metric is a metric computed based on data indicative of user pain acquired during each personal care session. For example, the metric could represent an average pain experienced during each personal care session. In other examples, it could represent a maximum pain level experienced during each session. In other examples, it could represent an average value of at least one sensor signal acquired over the session, for example application pressure of at least a portion of the device to bodily surfaces, where the sensor signal is taken as being a proxy for experienced pain. For example, it is known that application pressure can be expected to be inversely correlated with pain level, since a user presses less hard when they feel pain. For example, if the personal care device is an oral care device, e.g. a powered toothbrush device, the application pressure may correspond to application pressure of the brush head cleaning elements (e.g. bristles) to tooth/oral surfaces. Likewise, if the personal care device is grooming device, e.g. a shaver, a sensor signal may be indicative of application pressure of the shaver cutting area to the skin of the user during use. Such pressure sensor data provides information indicative of shaving action by the user over the course of one or more shaving sessions. Similar principles apply for other types of personal care device.

Different options for obtaining the data indicative of user pain will be described in greater detail later. In all cases, sensor data of some kind is acquired during the course of each personal care session and then used to infer information about the user's pain experience during the personal care session. Since, during the personal care session, the user is naturally probing or physically stimulating bodily surfaces (e.g. teeth) in a systematic way, the personal care session provides a convenient and efficient regularly occurring period where pain data can be acquired. Pain data is indicative of healing status of the relevant anatomy, e.g. mouth in the case of oral case.

In Fig. 3, the metric is computed for a period of four days prior to a treatment. The values of the metric are plotted on the graph. At day 5, the user/patient receives treatment, such as a dental operation, e.g. a root canal surgery, a dental extraction, or a filling. Following this the values of the metric significantly drop. This indicates that pain levels have increased, leading to a commensurate change in the metric. For example, the metric represents an inferred health status of the mouth. The values of the metric remain low for a period of approximately six days. Following this, the metric values recovery rapidly to their approximate value prior to the treatment and remain at this level for at least a further 15 days.

From this trending of the metric, a post-treatment recovery status can be computed. In some examples, one overall post treatment recovery status can be determined, based on the fact that the metric values returned to their pre-treatment level, this indicating a good post treatment recovery status. In some examples, a post-treatment recovery status could be computed at different time points following the treatment. In some examples, the post-treatment recovery status could be computed in part based on a time since the treatment and based on reference to an expected level of the metric, a proportion of the patient's pre-treatment level, given that time point following treatment. For example, the day following a surgery, the pain levels would typically be expected to be higher than before the surgery, and therefore the metric would be expected to be significantly lower than its value prior to the surgery. Therefore, a recovery status computed at this time might indicate normal recovery status, despite the fact that the metric values are significantly reduced. By contrast, at a time e.g. 10 days following the treatment, pain would be expected to have recovered to pre-treatment levels or better, and so likewise the value of the metric. Thus, a trended metric in which the metric values remain low for longer than a pre-defined threshold time period following treatment may indicate a poor recovery status.

Indeed, the latter scenario is illustrated in Fig. 4. This example has the same 4-day pre-treatment monitoring period, followed by the treatment. The metric values drop significantly, as in the scenario of Fig. 3, indicating elevated pain levels. However, in the scenario of Fig. 4, the metric values remain low for an extended period of time. Thus, in this scenario, the post-treatment recovery status after e.g. 10 days may indicate poor recovery.

The post-treatment recovery status could in some examples take one of a series of discrete classifications, e.g. slow recovery, normal recovery, fast recovery. The post-treatment recovery status could be computed as a numerical value, along a defined numerical scale.

Prior to a scheduled treatment, there may be a calibration monitoring period of e.g. 3-7 days in which the user pain data in each personal care session is acquired, and the metric computed for each personal care session based on this. As will be explained later, in a simple example, the pain data is obtained by measuring personal care device application pressure during each personal care session, and wherein the application pressure is taken to be inversely correlated with pain. The application pressure may be brushing pressure in the case of an oral care device. Then, for each personal care session, an overall pain level could be taken as the average pressure. The average pressure value for a given personal care session could be used as the value of the metric for that personal care session in a simple example.

Although an example has been presented above in which the recovery status is a post-treatment recovery status, in other examples, it could be a recovery status following a non-treatment event such as a consultation with a dentist or other practitioner at which no treatment is performed. For example, the user attends an appointment with a pain-related complaint and the practitioner advises the patient to simply wait and monitor the condition to see if it improves by itself.

Optionally, information indicative of a location on or in an anatomy at which a treatment was performed may be acquired, e.g. based on a user input by a clinical practitioner or by the user. For example, in the case of oral care, this may be information indicative of a location in the mouth at which a treatment was performed. In some examples, position sensing means may be part of the system, operable to track a position of an operative part of the personal care device on or in an anatomy to which the device is applied as a function of time, e.g. the brush head of a powered toothbrush, so that acquired pain data can be correlated to location on or in the anatomy (e.g. location in the mouth in the case of oral care). Optionally, if the bodily location to which the treatment corresponds is known (e.g. location in the mouth), then the pain data may be processed to extract only pain information relating to that location, and the metric computed and trended as a metric which corresponds only to that location.

After the treatment, the pain data is monitored during personal care sessions as before, and the metric computed as before. As stated above, optionally this may be limited to the particular location at which treatment was performed.

The metric is trended. Any (sudden) change from the pretreatment pattern of metric values is indicative of pain. If this change remains consistent, then pain is still there, and the patient is yet to recover. Duration (and optionally spread and intensity) of pain can optionally be extracted, and - depending on the procedure - the practitioner is provided insights to for example either call the patient for an appointment (if pain persists longer than normal) or keep monitoring (is progress is normal). Once the patient has returned to pretreatment metric values, this means pain has gone. The practitioner could even cancel a scheduled appointment if it is judged that the patient has fully recovered and a follow up is therefore no needed.

Thus, from the above example, it can be understood that determining the post-treatment or post-consultation recovery status may be based on: determining a baseline value for the metric, derived based on values of the metric corresponding to personal care sessions taking place prior to the treatment or the consultation; and trending values of the metric over time for personal care sessions following the treatment or consultation, and comparing the values of the metric following the treatment or the consultation to the baseline value. In some embodiments, the recovery status at a given point in time may comprise comparing the metric value at a given point in time against the baseline value, and then comparing any deviation against an expected or threshold deviation, and wherein the expected or threshold deviation may be a function of time following the treatment or consultation.

In a further embodiment, instead of the baseline being set based on the pre-treatment values of the metric, instead, the baseline can be set based on the values for an area of the relevant anatomy, e.g. the mouth, which is unaffected, e.g. an opposite side of the mouth to that which receives the treatment.

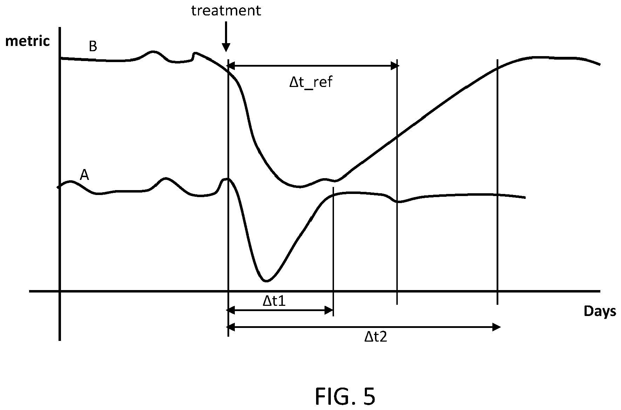

Fig. 5 illustrates a further example of trending values of the metric pre- and post-treatment or consultation. In this example, a time taken for metric values to return to a pre-treatment or pre-consultation level is used a simple indicator for recovery status. By way of example, computing the post-treatment recovery status may comprise determining, from trending of the metric over the series of personal care sessions, a time duration following the treatment or consultation for which the metric remains above a pre-determined threshold level. The method may further comprise comparing said time duration following treatment for which the metric values remain above a pre-determined threshold level against a pre-defined expected recovery time period, and determining whether the time period exceeds the expected recovery period. The metric could be a pain metric for example. The threshold metric value can be determined for example based on a pre-treatment metric value measured using sensor data from the at least one sensor acquired in advance of the treatment.

The idea in accordance with this example is for instance to classify a patient's healing rate for triaging purposes (e.g. to permit early recall for a follow-up consultation) and/or risk stratification to undergo in-office re-treatment.

Reference is made to Fig. 5 which illustrates tracking of post-treatment recovery rate, in order to assess post-treatment recovery status. Two scenarios are illustrated in Fig. 5, labelled scenario A and scenario B. Scenario A represents a rapid recovery rate, whereas scenario B represents a slower recovery rate. Each illustrated scenario shows a trend plot of computed values of the pain-derived metric for personal care sessions over time. As was discussed previously, the metric values are monitored over a period of time prior to a treatment and following a treatment. The timing of the treatment is indicated by an arrow in Fig. 5. The average metric value prior to treatment can be set as a baseline metric level. Following the treatment, the pain-derived metric values decline compared to the pre-treatment levels. The metric values are monitored over time following this.

From this trending, it can be determined a length of time taken for metric values to return to the baseline level, or a certain percentage of the baseline. The certain percentage could be defined in dependence upon the particular treatment or particular pathology which the patient is suffering from. The length of time taken for the metric values to recover to the pre-treatment levels can then be compared against a threshold or reference healing time. The threshold or reference healing time may be set in dependence upon the particular treatment or pathology. Based on whether the time taken for the metric values to rise to the baseline or defined proportion of the baseline is greater than the reference expected recovery time period, the recovery status can be classified, for example as fast, normal or slow healing.

For example, in the domain of oral care, after a treatment in the form of scaling and root planing, a patient can experience slight discomfort around the teeth for several days. One may notice some sensitivity to hot and cold (and sometimes sweets) for a duration of up to 6 weeks. It may take anywhere from 5-7 days for the gums to heal after a deep pocket cleaning or after a dental implant has been put in place. After implant surgery, it can take between 3-6 months for the jaw and gums to fully heal before the artificial tooth can be placed on the implant. Thus, depending upon the dental treatment, the expected normal recovery time can differ.

In the example of Fig. 5, the reference expected recovery time period is labelled as "• t_ref". The time period taken for the metric values to return to the pre-treatment baseline in scenario A is labelled • t1, and the time period taken for the metric values to return to the pre-treatment baseline in scenario B is labelled • t2. In this example, • t1 is less than • t_ref, and therefore, for the patient in scenario A, the recovery status is classified as 'fast recovery'. On the other hand, • t2 is greater than • t_ref, and therefore, for the patient in scenario B, the recovery status is classified as 'slow recovery'.

Depending on the classification, different response actions might be triggered. For example, if the recovery status is 'slow', then an appointment with the dental practitioner might automatically be scheduled, or the clinical practitioner might be alerted with an automatically generated alert message.

Additionally or alternatively, it is an option to explicitly compute a rate of change of the metric at one or more points in time. This rate of change can be compared against a threshold rate of change representing an expected rate of recovery, given the treatment. The expected rate of recovery at a given time point may also be a function of the time since the treatment took place. The threshold(s) can be stored in a look-up table for example.

Thus, in some embodiments, the determining the post-treatment recovery status comprises comparing a rate of change of said metric over at least a subset of the series of personal care sessions following treatment against a threshold rate of change.

As noted above, in accordance with any embodiments, the method may further comprise performing a response action based on the determined recovery status. This may comprise automatically scheduling an appointment or automatically transmitting an alert message to the clinical practitioner, e.g. dental practitioner.

Furthermore, as noted above, the method comprises generating a data report indicative of the recovery status. In accordance with one or more embodiments, the response action may comprise transmitting the data report via a communication channel to a practitioner computing device.

In some embodiments, the data report may be transmitted only in the event that the post-treatment recovery status is determined to be outside of a normal range, and optionally wherein the normal range is determined in dependence upon a time elapsed since the treatment event. In other words, if the recovery status is not normal, then the practitioner is alerted.

Part of the method relates to obtaining data indicative of user pain as a function of time over the course of each of a series of personal care sessions by a user using a personal care device, wherein the data indicative of user pain is determined based on sensor data from at least one sensor. Different approaches to obtaining the data indicative of user pain from different types of sensor will now be discussed.

In accordance with at least one set of embodiments, it is proposed to use physiological or biological sensor measurements. For example, skin conductance sensors, such as galvanic skin response (GSR) sensors, are known to provide an output which correlates with pain-related arousal. Thus a signal level as a function of time of a skin conductance sensor could in some embodiments be used as the pain signal as a function of time.

Another biological parameter measurement which is known to correlate with pain is heart rate variability. Reference is made for example to the paper: Forte, G., Troisi, G., Pazzaglia, M., Pascalis, V. D., & Casagrande, M. (2022). Heart Rate Variability and Pain: A Systematic Review. Brain Sciences, 12(2), 153.

Heart rate variability can be derived from a heart rate sensor signal, such as from a PPG sensor signal.

Another biological parameter measurement which is known to correlate with pain is breathing or respiration rate. Thus a respiration rate sensor could be used in some examples. For example, respiration rate can be derived from a PPG sensor signal.

Another biological parameter measurement which is known to correlate with pain is facial expression. Facial expression data could be obtained from processing of camera image data.

One or more biological sensors could be integrated in the personal care device itself. For example, a skin conductance sensor could be integrated in the handle portion of a personal care device, such as the handle portion of an oral care device, such as the handle portion of a powered toothbrush. Likewise, a PPG sensor could additionally or alternatively be integrated in the handle portion of a personal care device such as a shaver or powered toothbrush. Alternatively, a separate one or more biological sensing units could be provided. Alternatively, one or more biological sensors could be integrated in a wearable unit, such as a wristband or patch. In some embodiments, the processing device could also be integrated in the same wearable unit.

According to a further set of one or more embodiments, in addition to or instead of using biological sensor data to derive the data indicative of user pain as a function of time, data from force and/or motion sensors integrated in the personal care device may be used to infer pain information. In this case, the sensor data includes data from one or more sensors integrated in the personal care device indicative of personal care action by the user using the device over the course of one or more personal care sessions. These may include one or more force and/or motion sensors. Additionally or alternatively, data may be used from one or more operational signal sensors integrated in the personal care device adapted to output an operational signal indicative of an operational parameter of the device which correlates with physical interaction of the device with bodily surfaces. An example of an operational parameter is an electrical drive signal associated with an actuation device which actuates a portion of the personal care device, e.g. a drive train mechanism which drives oscillation of a powered toothbrush or shaver, or a motor mechanism which drives a massage device. The electrical characteristics of the drive signal can change as a function of applied pressure since applied pressure exerts more physical resistance on the actuation mechanism. Another example of an operational parameter is jet flow of an oral irrigation device or powered flossing device.

The information indicative of user pain during each of the one or more personal care sessions can be determined based on analysis of patterns in the sensor data.

The personal care device may be oral care device in some embodiments. The oral care device may be a powered toothbrush in some embodiments. The toothbrush may have a brush head carrying an array of cleaning elements such as bristles.

The integrated force and/or motion sensors may include a pressure sensor for sensing an application pressure of a portion of a personal care device to surfaces during use, e.g. of the cleaning elements of a brush head to teeth during operation. In the case of a toothbrush device, such a pressure sensor could be integrated in the brush head itself, but more usually would be integrated in the handle portion of the toothbrush, to which the brush head is attached. The brush head may be removably attached to the handle portion to enable replacement. Application pressure to teeth can be sensed e.g. via a cantilever pressure sensing approach.

The integrated force and/or motion sensors may include a motion sensor for sensing motion of the oral care device. The motion sensor may comprise or take the form of an inertial measurement unit (IMU) comprising typically one or more accelerometers, for example a tri-axial accelerometer arrangement.

There are different approaches to performing the analysis of patterns in the sensor data in order to determine the information indicative of user pain.

According to one approach, the obtained sensor data includes a pressure sensor waveform indicative of application pressure of a portion of a personal care device to bodily surfaces during use, and wherein the data indicative of user pain is determined based on the pressure signal. For example, if the personal care device is an oral care device in the form of a powered toothbrush device, the pressure sensor waveform may be indicative of application pressure of the brush head cleaning elements (e.g. bristles) to tooth/oral surfaces. Such pressure sensor data provides information indicative of cleaning action by the user using the device over the course of one or more oral cleaning sessions because it relates to how hard the user is pressing the cleaning elements onto the oral/dental surfaces during the cleaning session. Likewise, if the personal care device is grooming device, e.g. a shaver, the pressure sensor waveform may be indicative of application pressure of the shaver cutting area to the skin of the user during use. Such pressure sensor data provides information indicative of shaving action by the user over the course of one or more shaving sessions. Similar principles apply for other types of personal care device, a variety of examples of which have been discussed above.

Application pressure sensor data is useful for inferring data indicative of user pain during a personal cate session because it is known that the application pressure will be inversely related to the level of pain experienced (e.g. the brushing pressure reduces as the pain increases). Thus, analysis of the pattern of pressure over the course of a single personal care cycle (e.g. cleaning cycle), or multiple cycles, can provide information about user pain experience. For example, in a most simple embodiment, the observation of the pressure during the entire persona care cycle, e.g. brushing cycle, will show a pattern whereby differences in spread of application pressures as a function of time or location (for example compared to the baseline measurements) will provide an indication of pain: namely if unexpected drop in application pressure is seen in the pattern (where normally a fairly constant pressure would be observed), this may be an indication of a pain-causing abnormality in the lower-pressure area. Even more simply than this, a standard threshold pressure could be set, and wherein any drop in applied pressure below this threshold over a section of the sampled signal is taken as an indication of a pain experience in the location of the anatomy (e.g. mouth) to which that signal section corresponds.

In relation to this set of embodiments, as a general approach, it could be stated that the information indicative of user pain during each of the one or more personal care sessions can be determined further based on reference sensor pattern information associated with user pain or user comfort. The reference sensor pattern information could include a reference pattern of baseline brushing pressure as a function of time (or location) over the course of a personal care cycle, personalized to the user, and wherein pain in a personal care session is detected by comparing this reference baseline pattern to a pattern measured in the new personal care session. More simply, the reference sensor pattern information may include one or more characteristic pattern features in the sensor signal waveform which are each indicative of either a user pain reaction at a given moment during a personal care session, or user comfort during a given period of a personal care session. For the pressure sensor signal in particular, the relevant characteristic pattern feature would be a valley, or negative spike, in the pressure sensor waveform, indicative of a drop in applied pressure during a sub-interval of a given personal care session. For example, a valley in the signal in which the pressure falls below a pre-defined threshold baseline pressure could indicate a pain event for the sub-interval of the personal care session for which the pressure remains below that threshold. The threshold might be set according to a reference baseline pressure, for example acquired pre-treatment or otherwise during a period or at an anatomy location associated with low or zero pain.

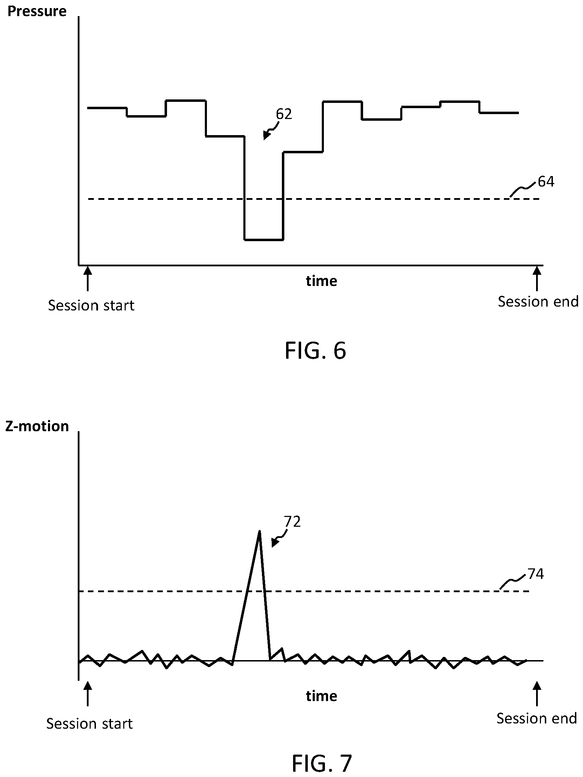

Fig. 6 illustrates an example in the context of an oral care device for performing a cleaning function. Fig. 6 illustrates an example brushing pressure signal as a function of time. A valley in the signal is indicated by arrow 62. During the sub-interval of time spanned by this valley, the applied pressure is below a defined threshold 64 and so it is determined that a pain event occurred during the sub-interval of time spanned by the valley. A valley 62 of depth lower than the threshold 64 represents a characteristic pattern feature whose detection is indicative of pain being experienced for the period of time spanned by the valley.

Optionally, in some embodiments, the pressure signal as a function of time or location in the mouth could be converted into a signal indicative of user pain as a function of time or location over the course of each of the one or more cleaning sessions. In a simple example, the pressure signal could be used directly as a proxy for pain, since it is expected to be inversely correlated with experienced pain.

As mentioned above, the brushing pressure signal would be rendered much more specific by addition of location information. The combination of a location sensor and a pressure sensor enables each pain event to be associated with an oral location. This allows a dentist or the user to know whether a pressure drop coincides with the position of a particular dental issue such as a post-treatment wound, whereby the signal can be attributed to pain level with greater certainty.

Thus, in some embodiments, wherein the obtained sensor data may include position sensor data, and wherein the method comprises determining information indicative of user pain as a function of location in the mouth. The determining the measure indicative of user pain as a function of location in the mouth may be based on using the position sensor data to correlate each of the detected valleys in the pressure signal to a corresponding position in the mouth. The method may furthermore comprise generating a pain map indicative of user pain as a function of location in the mouth and optionally communicating the generated pain map to a user interface for display on a user interface.

Furthermore, it is noted that the utility of a position sensor to correlate pain events with location in the mouth is not confined to embodiments which use a pressure sensor. Regardless of which particular force and/or motion sensor data is used to infer the pain information, the additional use of a location sensor allows characteristic pattern features associated with pain which are detected in the force/motion sensor signal to be correlated with a location in the mouth. Generation of a pain map, as suggested above, is also compatible more widely with any of the other described embodiments in this document.

In accordance with a further approach (which could be either combined with or used in place of the pressure-sensor approach already described), one or more motion sensing means can be used to sense motion of the personal care device. In this case, the sensor signal may show a sudden (impulse) movement of the personal care device as an acute pain is felt. The movement might be e.g. perpendicular to (e.g., away from) the anatomy surface (e.g. tooth surface), but sudden movement in any direction is possible following a feeling of pain. Here, the level of pain can be inferred as increasing as the motion impulse increases.

Thus, according to this approach, the sensor data may include a motion sensor signal, and wherein the information indicative of user pain is determined based on detecting inflections in motion, indicative of user reaction to locally experienced pain.

Thus this approach is also based on detecting one or more characteristic pattern features in the sensor signal waveform indicative of either a user pain reaction at a given moment during a personal care session, or user comfort during a given period of a personal care session. In this approach, the characteristic waveform pattern feature is an inflection or spike or impulse in the signal.

The motion sensor could include an inertial sensor such as an accelerometer for example.

Fig. 7 illustrates an example motion sensor signal as a function of time over the course of a single personal care session. The motion sensor in this case is sensitive at least to motion in a direction perpendicular (e.g. away from) the relevant anatomy surfaces (e.g. tooth surfaces) during use of the personal care device (denoted as the z-direction in Fig. 7). An upward inflection or spike or fluctuation in the signal is indicated at arrow 72. The amplitude or height of the inflection or spike or fluctuation in the signal exceeds a threshold motion level 74, which might in this instance correspond to a speed of motion or a rate of acceleration. If an inflection in the signal exceeds the pre-defined threshold 74 is may be classified as corresponding to a pain event.

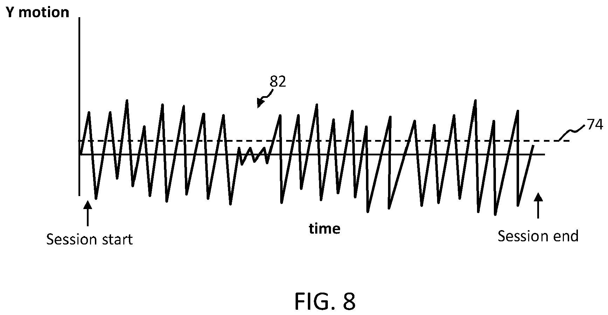

According to a further approach (which may be either combined with or used instead of any of the other approaches in this document), a motion sensor may be used which is operable to detect motion in one or more directions, e.g. during use of the personal care device. The patterns in this signal are representative of scrubbing action by a user, which manifests as an alternating pattern in the signal waveform. Fig. 8 shows an example. In this example, motion in a direction in-plane with the tooth surface (y-direction) is shown by way of illustration in the graph of Fig. 8. However, scrubbing motion could be sensed with a motion sensor sensing motion in any direction, e.g. simply a tri-axial accelerometer operable to sense motion in any direction.

At locations where pain is experienced, it is to be expected that amplitude of scrubbing motion would decrease compared to that in areas where no pain is felt. Thus here, the sensor data includes a motion sensor signal and wherein the information indicative of user pain is determined based on detecting in the motion sensor signal periods of alternating motion, indicative of a scrubbing action by the user, and wherein an amplitude or time duration of the periods of alternating motion are used to infer a level of patient comfort.

In particular, pain may be detected by detecting a characteristic waveform feature in the form of a section of an alternating signal having an amplitude which falls below a threshold 74. The threshold might be set in some examples as a defined proportion of an average amplitude of the signal over the whole of the personal session, for example half of the average amplitude. A sub-section of the signal during which the amplitude of a detected alternating motion signal is below the defined threshold may be classified as corresponding to pain events. Again, use of a location sensor in addition may allow these sub-sections of the signal to be correlated with particular locations of the anatomy, e.g. in the mouth, e.g. particular dental sites.

For example, the acquired sensor data can include position sensor data, and wherein the method comprises determining information indicative of user pain as a function of location on/in the anatomy, e.g. in in the mouth. User pain as a function of location may be determined based on using the position sensor data to correlate each of the detected signal periods of lower amplitude alternating motion to a corresponding position on/in the anatomy, e.g. in the mouth.

In addition to or instead of amplitude of the scrubbing motion, also frequency of scrubbing can be used to detect areas of higher pain. In particular, slow careful movements or scrubbing (at that location) are to be expected in areas of higher pain. Thus, in some embodiments, determining information indicative of user pain comprises determining a frequency of an alternating motion signal along a direction parallel with application surfaces (e.g. tooth surfaces), identifying continuous motion signal sections for which frequency is below a threshold frequency, and classifying those sections as correspond to pain events. Again, here the addition of a location sensor will improve specificity.

In addition to or instead of amplitude and frequency of the scrubbing motion, also the duration of time spent applying the device to a particular area, e.g. duration of time spent cleaning a particular tooth or dental area could be used to infer a level of pain experienced at that area. Here, the analysis of the sensor signal might comprise first identifying in a force and/or motion sensor signal a plurality of different signal sections corresponding to periods of time spent cleaning different areas, such as different dental areas, such as different teeth. In some cases, this could be done though correlating different signal sections with the output of a motion sensor. In other cases, it could be done through identifying pattern sections separated by breaks or pauses in the signal as the user moves to the next signal. For example for a pressure signal, the pauses might have a recognizable signature or fingerprint, such as being signal periods of a duration being below a threshold duration, and wherein for instance pressure or motion amplitude is below a certain threshold. Once the different signal sections have been identified, a time duration of each one can be determined, and from this it can be determined how long the user spent treating each area, e.g. cleaning each tooth. Dwell time on each tooth can alternatively be determined from an external source, such as from a brushing behavior monitoring module integrated in a native software application of the personal care device. A short time may indicate a higher pain. A short time combined also with presence during that signal section of one of the characteristic signal waveform features discussed, such as a lower amplitude alternating motion, or a spike in out-of-plane motion, or a dip in pressure, might be used to indicate pain.

Although the above example was described with reference to an example signal corresponding to in-plane motion, in practice the detected alternating motion can be in any direction.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing arrangement includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method for determining a status of a patient following a treatment or consultation, comprising:
obtaining data indicative of user pain as a function of time over the course of each of a series of personal care sessions by a user using a personal care device, the personal care sessions following the treatment or consultation, and wherein the data indicative of user pain is determined based on sensor data from at least one sensor;
determining a metric for each session based on the data indicative of user pain;
computing a post-treatment or post-consultation status based on trending of the metric over the series of personal care sessions following the treatment or consultation, and
generating a data report for export indicative of the computed post-treatment or post-consultation status.

2. The method of claim 1, wherein the at least one sensor is a sensor integrated in the personal care device.

3. The method of claim 1 or 2, wherein the sensor data includes data from a biological sensor.

4. The method of claim 3, wherein the biological sensor is a skin conductance sensor, such as a galvanic skin response (GSR) sensor.

5. The method of any of claims 1-4, wherein the sensor data includes data from one or more force and/or motion sensors integrated in the personal care device indicative of personal care action by the user using the device over the course of one or more personal care sessions, or from one or more operational signal sensors integrated in the personal care device adapted to output an operational signal indicative of an operational parameter of the device which correlates with physical interaction of the device with bodily surfaces.

6. The method of claim 5, wherein the information indicative of user pain during each of the one or more personal care sessions is determined based on analysis of patterns in the sensor data, and optionally based on reference sensor pattern information associated with user pain or user comfort.

7. The method of any of claims 1-6, wherein the computing the post-treatment or post-consultation status comprises determining, from trending of the metric over the series of personal care sessions, a time duration following the treatment for which the metric remains above a pre-determined threshold level.

8. The method of claim 7, wherein the method further comprises comparing said time duration following treatment for which the metric remains above a pre-determined threshold level against a pre-defined expected recovery time period, and determining whether the time period exceeds the expected recovery period.

9. The method of claim 7 or 8, wherein the threshold level is determined based on a pre-treatment or pre-consultation metric level measured using sensor data from the at least one sensor acquired in advance of the treatment.

10. The method of any of claims 1-9, wherein the determining the post-treatment or post-consultation status comprises comparing a rate of change of said metric over the series of sessions following treatment against a threshold rate of change.

11. The method of any of claims 1-10, further comprising performing a response action based on the patient status.

12. The method of any of claims 1-11, wherein the response action comprises transmitting the data report via a communication channel to a practitioner computing device.

13. The method of claim 12, wherein the data report is only transmitted in the event that the post-treatment or post-consultation status is determined to be outside of a normal range, and optionally wherein the normal range is determined in dependence upon a time elapsed since the treatment.

14. The method of any of claims 1-13, wherein the personal care device is an oral care device, the personal care session is an oral cleaning session and the personal care action is cleaning action.

15. A processing device, comprising:
an input/output; and
one or more processors, configured to:
obtain information indicative of user pain as a function of time over the course of each of a series of personal care sessions by a user using a personal care device, the personal care sessions following a treatment or consultation, and wherein the data indicative of user pain is determined based on sensor data from at least one sensor;
determine a metric for each session based on the data indicative of user pain;
compute a post-treatment or post-consultation status based on trending of said metric over the series of personal care sessions following the treatment or consultation, and
generate a data report for export indicative of the computed patient status.

16. A system comprising:
a personal care device comprising:
one or more force and/or motion sensors configured to generate sensor data indicative of personal care action by the user using the device over the course of one or more personal care sessions, or one or more operational signal sensors integrated in the personal care device adapted to output an operational signal indicative of an operational parameter of the device which correlates with physical interaction of the device with bodily surfaces; or
a biological sensor configured to generate sensor data indicative of a biological parameter of the user over the course of one or more personal care sessions; and
a processing device in accordance with claim 14, arranged to receive the generated sensor data as an input at the input/output.

17. The system of claim 16, wherein:
the processing device is integrated in the personal care device; or
the processing device is comprised by a computing device external to the personal care device, the computing device communicatively coupled with the personal care device or with a datastore with which the personal care device is communicatively coupled; or
the processing device is a cloud-based processing device.
